# EUROPEAN PATENT APPLICATION

(11) **EP 3 758 018 A1**
(43) Date of publication of application: **30.12.2020**
(21) Application number: 20182548.6
(22) Date of filing: 26.06.2020
(51) Int. Cl.: G16H 40/20, G06Q 50/22, G16H 50/30

(54) **RETRIEVAL APPARATUS, SYSTEM, METHOD, AND PROGRAM**

(30) Priority: 28.06.2019 JP 2019121818
(71) Applicant: TOYOTA JIDOSHA KABUSHIKI KAISHA, TOYOTA-SHI, AICHI-KEN 471-8571 (JP)
(72) Inventor: OTSUKI, Nobuhisa, Aichi-ken, 471-8571 (JP); NAKASHIMA, Issei, Aichi-ken, 471-8571 (JP); YAMAMOTO, Manabu, Aichi-ken, 471-8571 (JP)
(74) Representative: D Young & Co LLP

(57) **Abstract**

To retrieve job information appropriate to the body and mind functions of a person with disabilities, a retrieval apparatus (50) includes a storage unit (51) that stores job definition information (511) associating job information (5111) with required ability information (5112) indicating physical and mental ability required for a job, an acquisition unit (522) that acquires physical and mental ability information of a person with disabilities, a retrieval unit (523) that retrieves, as a potential job, the job information (5111) associated with the required ability information (5112) where the acquired physical and mental ability information is taken into consideration based on the job definition information (5111), and an output unit (525) that outputs presentation information based on the potential job.

## Description

### BACKGROUND

The present disclosure relates to a retrieval apparatus, system, method, and program.

Some people with disabilities use a rehabilitation support device when undergoing rehabilitation (rehab, training) in order to restore or maintain his/her body and mind functions. As an example of the rehabilitation support device, Japanese Unexamined Patent Application Publication No. 2015-223294 discloses a gait training device that includes a walking assist device which is to be worn on the leg of a client and assists them in walking.

### SUMMARY

People with disabilities are able to accomplish a job if the job tasks are adapted to his/her body and mind functions, and they can gain employment at a workplace where such kind of job is required. However, it is difficult to find an appropriate job where the body and mind functions of a person with disabilities are taken into consideration.

The present disclosure has been accomplished to solve the above problems and an object of the present invention is thus to provide a retrieval apparatus, system, method, and program for retrieving job information appropriate to the body and mind functions of a person with disabilities.

A retrieval apparatus according to a first aspect of the present disclosure includes a storage unit configured to store job definition information associating job information with required ability information indicating physical and mental ability required for a job, an acquisition unit configured to acquire physical and mental ability information of a person with disabilities, a retrieval unit configured to retrieve, as a potential job, the job information associated with the required ability information where the acquired physical and mental ability information is taken into consideration based on the job definition information, and an output unit configured to output presentation information based on the potential job.

According to the first aspect, it is possible to retrieve job information appropriate to the body and mind functions of a person with disabilities. It is thus possible to find an appropriate workplace that allows a person with disabilities to use his/her ability and thereby support his/her reintegration into society. Further, a company offering a job can employ a person with the required ability on the basis of job tasks.

The physical and mental ability information may contain a physical ability evaluation value based on a result of training performed by the person with disabilities to restore or maintain his/her physical ability by using a training device. Because the physical ability evaluation value is an objective determination index for the physical ability of a person with disabilities, it is possible to perform retrieval more appropriately.

The retrieval unit may derive deficiency of ability in the physical and mental ability information from the required ability information, identify supplementary information for supplementing the deficiency of ability, and retrieve the potential job with the identified supplementary information also taken into consideration in the physical and mental ability information, and the output unit may output the presentation information containing the potential job and the supplementary information. Thus, even if the current physical and mental ability information does not meet the physical and mental ability required for a job, it is possible to present a potential job at which a person with disabilities can gain employment by working with use of the supplementary information. This increases the choices of potential jobs.

The retrieval unit may identify, as the supplementary information, one or more assist devices that can supplement the deficiency of ability. It is thereby possible to search for a potential job for employment in the state where the physical and mental ability is improved using the assist device, which increases the choices of potential jobs.

The retrieval unit may identify, as the supplementary information, rehabilitation details for improving ability related to the deficiency of ability. It is thereby possible to search for a potential job for employment while improving the physical and mental ability by performing predetermined rehabilitation before the start of employment or in parallel with the start of employment, which increases the choices of potential jobs.

The retrieval unit may identify a rehabilitation facility that matches the identified rehabilitation details, and the output unit may output the presentation information to which the rehabilitation facility is added. A person with disabilities can thereby easily start training indicated by the identified rehabilitation details.

The storage unit may further store supplementary definition information associating each of a plurality of supplementary information with a supplementary value for improving physical and mental ability, and the retrieval unit may identify the supplementary information associated with the supplementary value that can supplement the deficiency of ability based on the supplementary definition information. It is thereby possible to objectively determine whether the deficiency of ability can be supplemented, and more accurately identify the supplementary information.

The acquisition unit may further acquire a dialogue history of the person with disabilities with a communication robot, and the retrieval unit may identify the supplementary information with the dialogue history also taken into consideration. It is thereby possible to more accurately retrieve a potential job that is appropriate to the cognitive function of a person with disabilities.

The retrieval apparatus may further include a calculation unit configured to calculate employment costs corresponding to the physical and mental ability information and the potential job, and the output unit may output the presentation information to which the employment costs are added. This enables more appropriate selection of a potential job.

The acquisition unit may further acquire public subsidy information related to employment of people with disabilities, and the calculation unit may calculate the employment costs with the public subsidy information also taken into consideration. An employer can thereby more appropriately determine the employment of a person with disabilities, which promotes the employment of people with disabilities based on the substantial labor costs.

The retrieval unit may identify, as the supplementary information, a plurality of types of assist devices that can supplement the deficiency of ability, the retrieval apparatus may further include a calculation unit configured to calculate a plurality of employment costs respectively corresponding to different combinations of the physical and mental ability information and each of the plurality of types of assist devices, and the output unit may output the presentation information to which the plurality of employment costs are added. This increases the choices of assist devices.

Working environment for a job may be further associated with the job information in the job definition information, and the retrieval unit may retrieve, as the potential job, the job information further associated with the working environment appropriate to the physical and mental ability information. This allows selection of a working environment more appropriate to the physical and mental ability of a person with disabilities, which increases the continuity of employment.

The output unit may output the presentation information whose disclosure range is varied depending on a receiver of disclosure. It is thereby possible to provide appropriate information for each receiver of disclosure and thereby allow each receiver to effectively use the information.

The retrieval apparatus may further include a billing unit configured to, when the person with disabilities gains employment at the potential job contained in the presentation information, bill a provider of the information contained in the presentation information. It is thereby possible to ensure the revenues of a job search service for people with disabilities.

The retrieval apparatus may further include a registration unit configured to, when the person with disabilities gains employment at the potential job contained in the presentation information, register record information associating this presentation information with the acquired physical and mental ability information into the storage unit, and the retrieval unit may retrieve the potential job with the record information also taken into consideration. It is thereby possible to more accurately retrieve a potential job for a person with disabilities having the physical and mental ability information similar to that in a record of past employment.

A retrieval system according to a second aspect of the present disclosure includes a storage device configured to store job definition information associating job information with required ability information indicating physical and mental ability required for a job, and a retrieval apparatus including an acquisition unit configured to acquire physical and mental ability information of a person with disabilities, a retrieval unit configured to retrieve, as a potential job, the job information associated with the required ability information where the acquired physical and mental ability information is taken into consideration based on the job definition information, and an output unit configured to output presentation information based on the potential job.

A retrieval method according to a third aspect of the present disclosure includes, in a computer, acquiring physical and mental ability information of a person with disabilities, based on job definition information associating job information with required ability information indicating physical and mental ability required for a job, retrieving, as a potential job, the job information associated with the required ability information where the acquired physical and mental ability information is taken into consideration, and outputting presentation information based on the potential job.

A retrieval program according to a fourth aspect of the present disclosure causes a computer to execute acquiring physical and mental ability information of a person with disabilities, based on job definition information associating job information with required ability information indicating physical and mental ability required for a job, retrieving, as a potential job, the job information associated with the required ability information where the acquired physical and mental ability information is taken into consideration, and outputting presentation information based on the potential job.

According to the second, third and fourth aspects also, the same effects as in the first aspect can be obtained.

According to the present disclosure, it is possible to provide a retrieval apparatus, system, method, and program for retrieving job information appropriate to the body and mind functions of a person with disabilities.

The above and other objects, features and advantages of the present disclosure will become more fully understood from the detailed description given hereinbelow and the accompanying drawings which are given by way of illustration only, and thus are not to be considered as limiting the present disclosure.

### BRIEF DESCRIPTION OF DRAWINGS

Fig. 1 is a block diagram showing the overall configuration of a retrieval system according to a first embodiment;
Fig. 2 is a block diagram showing the configuration of a retrieval apparatus according to the first embodiment;
Fig. 3 is a view showing an example of job definition information according to the first embodiment;
Fig. 4 is a view showing an example of supplementary definition information according to the first embodiment;
Fig. 5 is a flowchart showing the flow of a retrieval method according to the first embodiment;
Fig. 6 is a block diagram showing the configuration of a retrieval apparatus according to a second embodiment;
Fig. 7 is a flowchart showing the flow of a retrieval method according to the second embodiment;
Fig. 8 is a block diagram showing the overall configuration of a retrieval system according to a third embodiment;
Fig. 9 is a block diagram showing the configuration of a retrieval apparatus according to the third embodiment;
Fig. 10 is a flowchart showing the flow of a billing process according to the third embodiment;
Fig. 11 is a flowchart showing the flow of a record registration process according to the third embodiment;
Fig. 12 is a flowchart showing the flow of a retrieval method according to the third embodiment; and
Fig. 13 is a block diagram showing the overall configuration of a retrieval system according to a fourth embodiment.

### DESCRIPTION OF EMBODIMENTS

Specific embodiments of the present disclosure including the above-described aspects will be described hereinafter in detail with reference to the drawings. The same or corresponding elements are denoted by the same symbols throughout the drawings, and duplicated explanations are omitted as necessary for the sake of clarity.

### <First Embodiment>

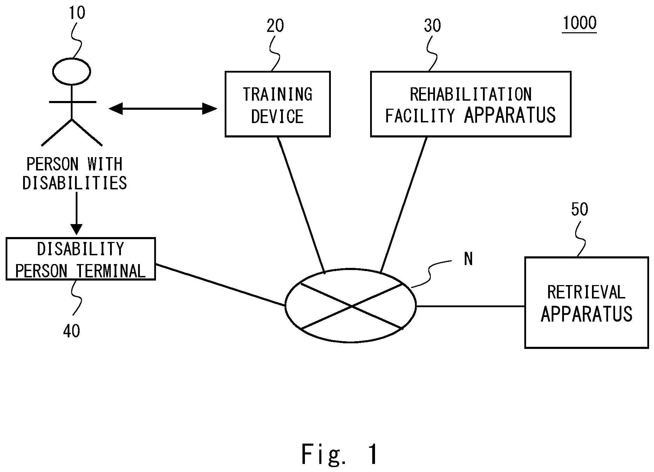

Fig. 1 is a block diagram showing the overall configuration of a retrieval system 1000 according to a first embodiment. The retrieval system 1000 includes a training device 20 to be used by a person 10 with disabilities, a rehabilitation facility apparatus 30, a disability person terminal 40, and a retrieval apparatus 50. The training device 20, the rehabilitation facility apparatus 30, the disability person terminal 40 and the retrieval apparatus 50 are connected for communication through a network N. The network N is a communication network such as the Internet, an intranet, a mobile telephone network or a LAN (Local Area Network).

A person 10 with disabilities is a person who is subject to limitations in his/her life due to physical or mental disabilities and who is aiming at reintegration into society (for example, gaining a job) regardless of having physical or mental disabilities. In the example of Fig. 1, the person 10 with disabilities is a person who undergoes training in order to restore or maintain his/her physical ability by using the training device 20, etc. in a rehabilitation facility such as a medical institution. The person 10 with disabilities is, for example, a rehabilitation patient who undergoes rehabilitation in order to restore the physical ability after surgery performed because of suffering from a serious illness or a severe injury and who remains mentally or physically impaired compared with a person without disabilities even after rehabilitation. Alternatively, the person 10 with disabilities may be an elderly person, etc. who is mentally or physically impaired and undergoes rehabilitation in order to maintain the physical and mental ability. The rehabilitation is a training process to be performed in order to restore or maintain the physical function or the intellectual function, or reduce the mental or physical disability, regardless of whether it is covered by insurance or not, or whether medical equipment is used or not. Note that the person 10 with disabilities does not necessarily perform rehabilitation or, even if rehabilitation is performed, there is no need to use the training device 20. The person 10 with disabilities may be a person whose physical and mental ability information at least indicating his/her physical and mental ability, which is described later, is stored in some information processing apparatus.

The training device 20 is a device for the person 10 with disabilities to undergo training with the assistance of a training staff by instructions or helping in order to restore or maintain his/her physical and mental ability. The training device 20 may be a rehabilitation support device such as the gait training device disclosed in Japanese Unexamined Patent Application Publication No. 2015-223294, for example, though not limited thereto.

In this case, it is assumed that the training device 20 acquires detection data detected (measured) by an internal or external sensor, etc. in the course of rehabilitation of the person 10 with disabilities. Note that the detection data is an example of the physical and mental ability information, and it may be a set of measured values respectively corresponding to a plurality of indices (for example, a plurality of sensors) for measurement. Further, the detection data is data where a measured value is associated with measurement time and an index.

The detection data is, mainly, sensor data. The sensor data is sensor values detected by the sensors (not shown) in the training device 20. For example, the sensor data may be the tilt angle of the trunk detected by a posture sensor, the load or the slope angle detected by a handrail sensor, the angle detected by an angle sensor, and so on. The sensors that output the sensor data are an acceleration sensor, an angular velocity sensor, a position sensor, a light sensor, a torque sensor, a weight sensor, and so on. Further, an encoder included in a motor of a winding structure, etc. for wires of the training device 20 may be used as a sensor. Furthermore, a torque sensor (load cell) of a motor may be used as a sensor, or a current detector that detects a drive current value for driving the motor may be used as a sensor.

The sensor data may contain line-of-sight data acquired by a line-of-sight sensor that detects the line of sight, for example. Such line-of-sight data may be also obtained by detecting the line of sight from image processing based on an image at least showing the eyes of the person 10 with disabilities or obtained by determining the orientation (looking up, looking down, etc.) of the face based on an image at least showing the face of the person 10 with disabilities. Such data may be contained in the above-described detection data. Further, the detection data may be audio data (voice data) acquired by a voice acquisition unit such as a microphone that acquires the voice of the person 10 with disabilities or a training staff, text data obtained by analyzing this voice data, or data obtained by analyzing this text data. The voice of a training staff may contain the voice talked to the person 10 with disabilities for correcting the gait, etc. Furthermore, the sensor data may be detection data of the brain wave of the person 10 with disabilities detected by an electroencephalograph, or detection data of the brain wave of a training staff detected by an electroencephalograph.

While the line-of-sight sensor, an imaging unit that takes the above-described image, the microphone and so on may be mounted on the main body of the training device 20, they may be mounted on a glasses-type wearable terminal to be worn by the person 10 with disabilities, for example. This terminal is equipped with a wireless communication unit that performs wireless communication of data by wireless communication technology such as Bluetooth (registered trademark), and the training device 20 may be also equipped with a wireless communication unit. The training device 20 can thereby acquire data acquired by the wearable terminal by wireless communication. The electroencephalograph may be mounted on the main body of the training device 20 and configured to be able to detect the brain wave of the person 10 with disabilities and the brain wave of a training staff separately from each other, though high detection accuracy is needed in this case. The electroencephalograph, however, is preferably mounted at the position where it comes into close proximity with a target person, such as on the above-described glasses-type wearable terminal (for example, on the temple of glasses).

Further, a sensor, etc. that detects the detection data is not limited to those described above. For example, the person 10 with disabilities may wear clothing with a clothing-embedded biosensor and/or a clothing-embedded touch sensor. The clothing is not limited to upper body clothing, and they may be lower body clothing or a set of upper and lower body clothing, or one to be worn on a part of the body, such as a harness, for example. Further, clothing and the training device 20 are equipped with a wireless communication unit as described above. The training device 20 can thereby acquire data acquired by the clothing-embedded biosensor or the clothing-embedded touch sensor by wireless communication.
The clothing-embedded biosensor can acquire vital data such as the heart rate of a person wearing it. The clothing-embedded touch sensor can acquire information where the person 10 with disabilities who is wearing it is touched from the outside, which is data indicating information about the position where a training staff touches the person 10 with disabilities.

The detection data may further contain values calculated based on detection signals from a plurality of sensors or statistics obtained from statistical processing of detection signals from one or a plurality of sensors and so on, not limited to values indicated by the detection signals detected by the sensors. The statistics may be various statistics such as the average value, the maximum value, the minimum value and the standard deviation value, for example, and they may be static statistics or dynamic statistics during a certain period of time such as one day, one training or one gait cycle, for example.

For example, the sensor data may contain the knee joint opening angle calculated from the angle between an upper leg frame and a lower leg frame detected by the angle sensor. Further, the sensor data regarding the angle sensor may contain the angular velocity obtained from the differentiation of the angle. The sensor data regarding the acceleration sensor may be the velocity obtained from the differentiation of the acceleration or the position obtained from performing the differentiation twice.

For example, the detection data may contain the average value, the total value, the maximum value, the minimum value and the representative value as follows for each day, or each rehabilitation training in one day. The average value may be the average speed (total walking distance/total walking time) [km/h], the average value of the stride length [cm], the walking rate [steps/min] indicating the number of steps per minute, the walking PCI [beat/m], fall prevention help [%], and so on. The average speed may be a value calculated from the speed set value of the treadmill or a value calculated from a drive signal in a treadmill drive unit, for example. The stride length is the distance from ground contact of one heel to the next ground contact of the same heel. The PCI stands for Physiological Cost Index, and the walking PCI indicates the energy efficiency during walking. The fall prevention help [%] indicates the percentage that calculates the fall prevention helps [times] indicating the number of times a training staff gives the fall prevention help for the person 10 with disabilities per step, which is the percentage of performing fall prevention help per step.

The total value may be the walking time [seconds], the walking distance [m], the number of steps [steps], the fall prevention help [times], the body part and the number of times per body part [times] for which the fall prevention help is given, and so on.

The maximum value or the minimum value in this example may be the maximum value or the minimum value of the continuous walking time [seconds], the continuous walking distance [m], the successive number of steps [steps], and so on, the minimum value of the walking PCI [beat/m] (in other words, the longest distance the person can walk per beat), and so on. The representative value may be the value used most frequently as the speed of the treadmill (representative speed [km/h]).

As described above, data supplied directly or indirectly from detection units such as sensors may be contained in the detection data. Further, time information such as the date and time when data is detected or timing information different from time may be added to the detection data.

Note that the above-described detection data are just examples, and other detection data may be used. Alternatively, some of the above-described detection data may be not used.

The physical and mental ability information may contain index data containing at least one of the symptom, the physical ability and the recovery level of the person 10 with disabilities regarding the rehabilitation performed by the person 10 with disabilities by using the training device 20. In other words, the physical and mental ability information may contain the symptom information, the Br. stage, the SIAS, the initial gait FIM, the latest gait FIM, and so on of the person 10 with disabilities. Note that the physical and mental ability information may contain various data indicating the physical ability of the person 10 with disabilities. Further, such index data of the person 10 with disabilities may be determined or calculated in the training device 20, or may be evaluated by a training staff such as a doctor or a physical therapist, input through an input device (not shown), and stored into a storage device in the training device 20. Further, the physical and mental ability information may contain harness information about a harness used by the person with disabilities.

The symptom information may contain information indicating an initial symptom, the time of emergence of the symptom, and the current symptom, and it can be considered that the person 10 with disabilities is in need of rehabilitation mainly due to the symptom contained in this information. Note that, however, a symptom that is not likely to be directly related to the rehabilitation may be also contained in the symptom information. Further, the symptom information may contain the type of disease (disease name or disorder name) the person with disabilities is affected, such as stroke (cerebrovascular disease) or spinal cord injury, and the affected area (the area of injury), and in some disease type, may contain their category. For example, stroke may be classified into cerebral infarction, intracranial hemorrhage (brain hemorrhage/subarachnoid hemorrhage), and so on.

The Br. stage, which indicates Brunnstrom Recovery stage, divides the process of recovery from hemiplegia into six stages based on observation. The disability person data may contain the items in the lower extremity section, which are main items related to the training device 20. The SIAS stands for Stroke Impairment Assessment Set, which is an index for comprehensively evaluating the dysfunction caused by a stroke. The SIAS may contain hip-flexion test (Hip-Flex), knee-extension test (Knee-Ext), and foot-pat test (Foot-Pat). The SIAS may also contain lower extremity light touch (Touch L/E), lower extremity position sense (Position L/E), abdominal muscle strength (Abdominal), and verticality test (Verticality).

The FIM (Functional Independence Measure) defines one of evaluation methods for ADL (Activities of Daily Life). In the FIM, each item is rated on a 7-point scale based on the level of assistance.

For example, the gait FIM is a universal index indicating the level of recovery. When a person with disabilities can walk 50 m or longer without helper and harness (assistive device), the score is 7 points, which is highest. When, on the hand, the person with disabilities can only walk less than 15 m with the assistance of one helper, the score is 1 point, which is lowest. When the person with disabilities can move 50 m with the minimal assistance (the level of assistance = less than 25%), the score is 4 points, and when the person with disabilities can move 50 m with the moderate assistance (the level of assistance = 25% or more), the score is 3 points. Thus, as the recovery progresses, the gait FIM of the person 10 with disabilities increases accordingly.

As described above, the latest gait FIM employed in the training device 20 serves as an index indicating the physical ability of the person 10 with disabilities and also as an index indicating the level of recovery of the person 10 with disabilities from the start of rehabilitation. Thus, the gait FIM is an important index in terms of knowing the progress of the rehabilitation of the person 10 with disabilities. Further, the amount of change or the rate of change from the initial gait FIM to the latest gait FIM also serves as an index indicating the level of recovery. The rate of change may be referred to also as FIM efficiency, and it may be the value obtained by dividing the gain (the amount of change) of FIM up to the present by the number of days rehabilitation is performed, the number of days elapsed indicating the period of rehabilitation, or the length of hospital stay when the person 10 with disabilities is a hospitalized patient, for example.

Further, the gait FIM may be regarded as a score under certain conditions at the time of evaluation, such as when adaptive equipment is worn, and in this case, information indicating the conditions applied at the time of evaluation may be added to information indicating the gait FIM. The conditions may contain a raise height, a harness used (for example, a walking assist device, without a harness etc.), the setting of the angle of a knee or ankle part of this harness, etc. whether level-ground walking or slope walking, and so on, when this information is acquired. Further, the gait FIM is typically a gait FIM in level-ground walking, and level-ground walking information indicating it may contain information such as the longest distance (maximum continuous walking distance [m]) at the time of level ground walk evaluation.

The data, such as the latest gait FIM, that can be included in two concepts of the physical ability and the recovery level may be generally included in one of them; however, it may be included in both of them.

The training device 20 may associate disability person attribute information such as the attributes, the physical information, and so on of the person 10 with disabilities with the above-described physical and mental ability information and store them into an internal storage device. The disability person attribute information may contain the age, the gender, the physical size (body height, weight, etc.), and so on of the person 10 with disabilities. Further, the disability person attribute information may contain the name or ID of the person 10 with disabilities, and the disability person attribute information may also contain preference information indicating the preference of the person 10 with disabilities, personality information indicating the personality of the person 10 with disabilities, and so on. Furthermore, the disability person attribute information may contain, as FIM, exercise items different from those related to the ability to walk, and it may also contain cognitive items. Note that a part or whole of the physical and mental ability information or the disability person attribute information may be referred to as physical information, basic information, or disability person feature information, and so on. The training device 20 may acquire the index data, the disability person attribute information and so on from an electronic health record system (not shown) of a medical institution, etc. or by input of a training staff.

Further, the training device 20 may associate the disability person attribute information, etc. with the physical and mental ability information, such as detection data and index data, and store them into a storage device in the training device 20. Alternatively, the training device 20 may transmit the physical and mental ability information, etc. to the rehabilitation facility apparatus 30 through the network N, and store them into a storage device in the rehabilitation facility apparatus 30. Alternatively, the training device 20 may transmit the physical and mental ability information, etc. to the retrieval apparatus 50 through the network N in response to operation of a training staff, etc., detection of detection data, a predetermined interval, or an acquisition request from the retrieval apparatus 50.

The rehabilitation facility apparatus 30 is an information processing apparatus that is installed in a facility where the person 10 with disabilities undergoes rehabilitation by using the training device 20, such as a medical institution, or a facility that manages this rehabilitation, or that is at least administered by this facility. The rehabilitation facility apparatus 30 is a database system that at least manages the physical and mental ability information and the like.

For example, the rehabilitation facility apparatus 30 receives the physical and mental ability information and the disability person attribute information acquired from the training device 20 through the network N, and stores the physical and mental ability information and the disability person attribute information in association with each other into an internal storage device. Further, the rehabilitation facility apparatus 30 may acquire the detection data from the training device 20 through the network N, and acquire the index data, the disability person attribute information, and so on from an electronic health record system (not shown) of a medical institution, etc., or by input of a training staff. In the case where the person 10 with disabilities performs training using a device different from the training device 20, the rehabilitation facility apparatus 30 may acquire data equivalent to the above-described data by input of a training staff.

Further, the rehabilitation facility apparatus 30 may transmit the physical and mental ability information, etc. to the retrieval apparatus 50 through the network N in response to operation of a training staff, etc., acquisition of the physical and mental ability information, a predetermined interval, or an acquisition request from the retrieval apparatus 50. Note that the configuration of the rehabilitation facility apparatus 30 can be implemented by a known information system, etc., and the detailed description thereof is omitted.

The disability person terminal 40 is an information processing apparatus to be used by a user on the disability person side, including the person 10 with disabilities, and a family member, a guardian, etc. of the person 10 with disabilities. The disability person terminal 40 has a communication function through the network N, and it may be a personal computer, or a portable information terminal such as a tablet terminal or a smartphone, for example. The disability person terminal 40 receives a search request (job search request) to retrieve a workplace (job) for employment through an input device in response to a user's operation on the disability person side, and transmits the job search request to the retrieval apparatus 50 through the network N. Further, the disability person terminal 40 receives a search result from the retrieval apparatus 50 through the network N, and outputs (displays etc.) the result to an output device such as a screen.

The retrieval apparatus 50 is one or more information processing apparatuses for the person 10 with disabilities under or after rehabilitation to search for a workplace (job) for employment that matches his/her physical and mental ability after training. The retrieval apparatus 50 is a server device, for example, and it is assumed that a web server, an application server and a database server are running on an OS (Operating System), and a web application that implements retrieval processing according to this embodiment is running on the application server. Note that, however, the software configuration of the retrieval apparatus 50 is not limited thereto.

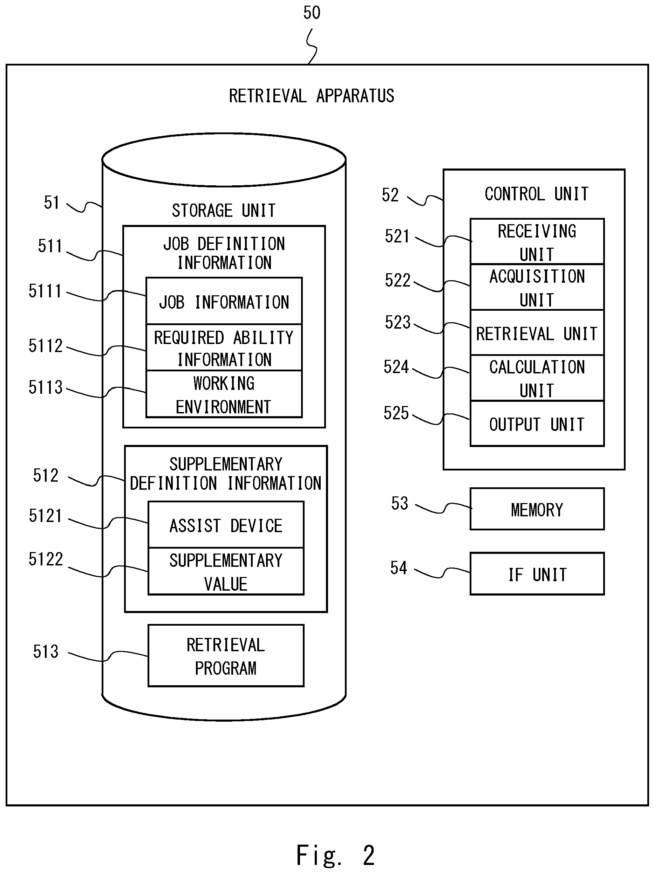

Fig. 2 is a block diagram showing the configuration of the retrieval apparatus 50 according to the first embodiment. Fig. 2 shows an internal configuration in the case where the retrieval apparatus 50 is configured using one computer device in the form of a functional block.

The retrieval apparatus 50 includes a storage unit 51, a control unit 52, a memory 53, and an IF (InterFace) unit 54. The storage unit 51 is a non-volatile storage device such as a hard disk or a flash memory, for example. The storage unit 51 at least stores job definition information 511, supplementary definition information 512, and a retrieval program 513.

The job definition information 511 is information that associates job information 5111, required ability information 5112, and working environment 5113. The working environment 5113 is not essential, and the job definition information 511 may be information that at least associates the job information 5111 with the required ability information 5112. For example, the job definition information 511 may be a conversion table or a conversion map that defines conversion rules from the required ability information 5112 to the job information 5111 (and the working environment 5113), for example. In this case, the job definition information 511 may contain a plurality of conversion tables for different types of required ability information 5112. Alternatively, the job definition information 511 may be a converter that performs conversion from the required ability information 5112 to the job information 5111 (and the working environment 5113), a function that inputs the required ability information 5112 and outputs the job information 5111 (and the working environment 5113), a conversion algorithm, or a learned conversion model.

The job information 5111 is identification information of a job, text information indicating tasks or work items of a job, and so on. Further, a job is a job category, tasks, and so on in a specific workplace, regardless of whether a contract of employment is made or not. Furthermore, the job information 5111 may contain information about a company offering this job, etc. The information about a company, etc. is the name of a company, the address of the place of work, the nearest station, conditions of a contract of employment, and so on, for example.

The required ability information 5112 is information indicating the physical and mental ability required for a job. The required ability information 5112 may be information similar to the above-described physical and mental ability information, for example. The required ability information 5112 contains a measured value (a result of training) measured when the person 10 with disabilities has performed training using the training device 20. The result of training is the detection data or the sensor data detected by the training device 20 described above, for example, and it may be a set of measured values by a plurality of indices. Further, the training includes gait training, trunk balance (balance training), exercises to prevent dementia, and so on, and, in this case, a result of such training is contained in the required ability information 5112.

Further, the required ability information 5112 contains an index value of the physical ability during and after training of the person 10 with disabilities, or a physical evaluation value determined based on a result of training. The physical evaluation value contains the above-described Br. stage, SIAS, initial gait FIM, latest gait FIM, and so on, for example.

The working environment 5113 is information indicating the workplace to engage in a job corresponding to the job information 5111, the indoor and surrounding environment, and so on. The working environment 5113 is, for example, information about the seating (desk size, chair shape), information about around the seating, route information such as the length and width of the route between the entrance and the seating, route information including the route, stairs, elevator, slope, handrail, and so on, from the entrance of the building to the place of work, route information about the route between the place of work and a toilet, a company store, and so on. Further, the working environment 5113 may contain information indicating the barrier-free level of the indoor and surrounding environment.

Note that the job definition information 511 may be generated by receiving, from a company that has employed a person with disabilities, past record information such as tasks assigned to this person, the working environment of this company, and the physical and mental ability information of this person, or know-how.

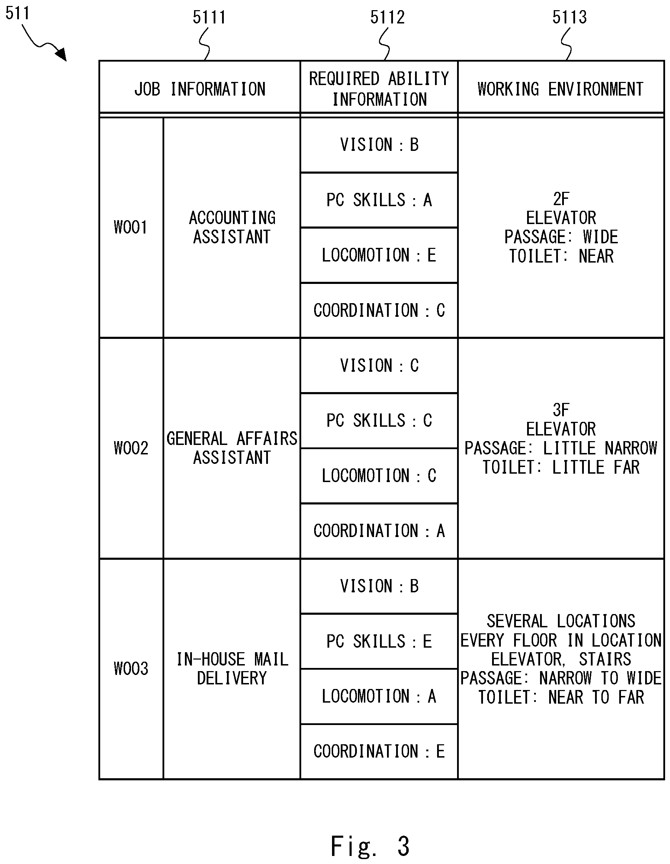

Fig. 3 is a view showing an example of the job definition information 511 according to the first embodiment. The job information 5111 is a job ID and a job name, the required ability information 5112 is a set of levels required for a job for each type of physical and mental ability, and the working environment 5113 is a floor number of the place of work, the presence or absence of an elevator, the width of a passage, the distance from a toilet, and so on. Note that, although the required ability information 5112 is ranked in five levels from A to E, from highest to lowest, in the example of Fig. 3, this is not limited thereto. For example, the required ability information 5112 may represent, in numerical values, the level required for a job for each type of physical and mental ability.

Further, the required ability information 5112 may be a pair of physical evaluation values such as the FIM and the SIAS. For example, the gait FIM and the SIAS, which is a distal lower extremity test, have a correlation as an index of the ability to walk (the ability to move). Therefore, a combination of a value of the gait FIM and a value of the SIAS, which is a distal lower extremity test, may be used as an index value indicating the level of walking ability required for the job.

Further, in the FIM, middle classification items such as self-care, sphincter control, transfers and locomotion belong to the exercise items, and a plurality of small classification items belong to each of the middle classification items. Further, in the FIM, middle classification items such as communication and social cognition belong to the cognitive items, and a plurality of small classification items belong to each of the middle classification items. Note that, however, a plurality of small classification items that belong to different middle classification items can include the same motion. For example, the small classification item "bed, chair, wheelchair" that belongs to the middle classification item "transfers" and the small classification item "walk, wheelchair" that belongs to the middle classification item "locomotion" have high correlation of motion. Therefore, a combination of the FIM of the small classification item "bed, chair, wheelchair" and the FIM of the small classification item "walk, wheelchair" may be used as the ability to walk among the required ability information 5112.

Referring back to Fig. 2, the supplementary definition information 512 is an example of information that associates each of a plurality of supplementary information with a supplementary value for improving the physical and mental ability. The supplementary information is information that can supplement an ability value indicating the gap between the physical and mental ability information of the person 10 with disabilities and the required ability information 5112 (the deficiency of ability). The supplementary definition information 512 according to this embodiment associates an assist device 5121 with a supplementary value 5122. The assist device 5121 is an example of the supplementary information. The assist device 5121 is, for example, a wearable device that supports hearing, seeing or cognitive function, smartglasses that support seeing or cognitive function, a cane that supports the ability to walk (the ability to move), a mobility such as a wheel chair or a personal mobility, an assistant robot that supports the cognitive function, and so on, though not limited thereto. Supporting the cognitive function includes displaying text indicating work instructions on smartglasses, outputting work instructions by sound from a wearable device or an assistant robot that supports hearing, or speaking and giving advice on work by an assistant robot, and so on. Further, the supplementary value 5122 is a level or an index value when the physical and mental ability increases if the person 10 with disabilities wears, uses, etc. the associated assist device 5121.

Note that the supplementary definition information 512 may be generated by receiving, from a company that has employed a person with disabilities, past record information such as tasks assigned to this person, the physical and mental ability information of this person, and the assist device used by this person, or know-how.

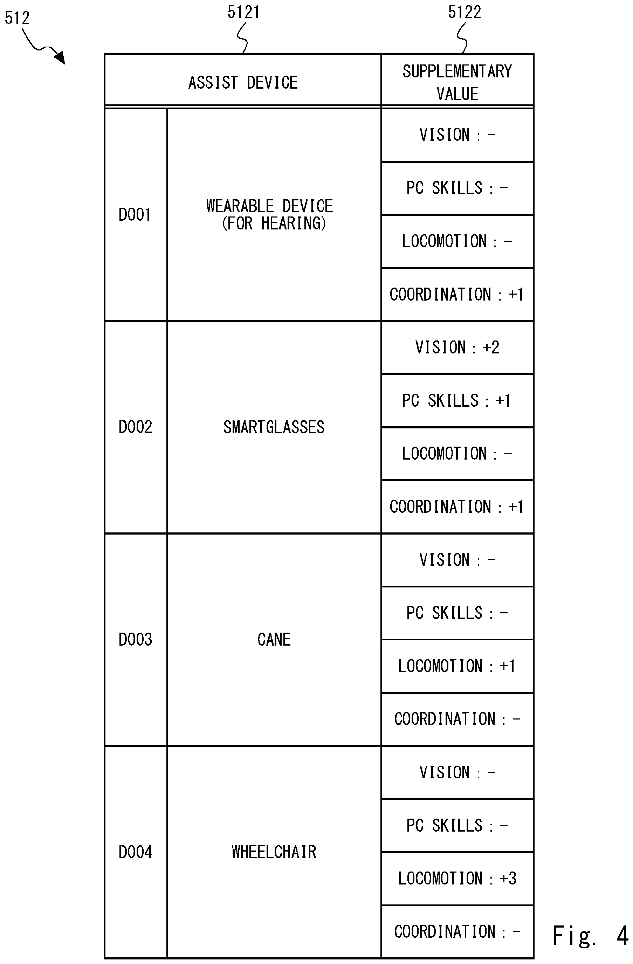

Fig. 4 is a view showing an example of the supplementary definition information 512 according to the first embodiment. The assist device 5121 is a device ID and a device name, the supplementary value 5122 is an example of an increased value of the physical and mental ability level for each type of the physical and mental ability. For example, in the case of smartglasses with the device ID "D002", when the person 10 with disabilities wears them and carries out a job, the current level of vision of the person 10 with disabilities increases by two, and (with the increase in vision), the level of PC skills increases by one, and the level of ability to coordinate things increases by one. The supplementary value 5122 is not limited to numerical values.

Referring back to Fig. 2, the retrieval program 513 is a computer program that performs processing for a retrieval method according to the first embodiment.

A memory 53 is a non-volatile storage device such as a RAM (Random Access Memory), and it is a storage area for temporarily storing information during operation of a control unit 52.

An IF unit 54 is an interface that inputs and outputs information to and from the outside of the retrieval apparatus 50. The IF unit 54 is a communication circuit for at least performing communication through the network N.

The control unit 52 is a processor that controls each component of the retrieval apparatus 50. The control unit 52 loads the retrieval program 513 from the storage unit 51 to the memory 53 and executes the retrieval program 513. The control unit 52 thereby implements the functions of a receiving unit 521, an acquisition unit 522, a retrieval unit 523, a calculation unit 524, and an output unit 525, which are described later.

The receiving unit 521 receives a search request (job search request) to retrieve a workplace (job) for employment for the person 10 with disabilities. For example, the receiving unit 521 receives the job search request that contains job tasks desired by the person 10 with disabilities as search condition from the disability person terminal 40 through the network N. The search condition may be a type of industry, specific job tasks, working hours, the number of days available per week, a place of work, a nearest station, conditions of a contract of employment and so on, for example, though not limited thereto. Further, the search condition may contain needs regarding action during work or needs for the working environment by the person 10 with disabilities. The needs regarding action may be, for example, that the person 10 with disabilities desires to move on his/her own or move using an assist device in the working environment. Thus, the needs regarding action may contain whether there is a request for an assist device, such as a harness, not currently worn. Further, the needs for the working environment may be, for example, the distance from the place of work to a toilet. The receiving unit 521 may further receive input information of physical information of the person 10 with disabilities. The physical information is the above-described disability person attribute information, for example.

The acquisition unit 522 acquires the physical and mental ability information of the person 10 with disabilities. For example, the acquisition unit 522 acquires the physical and mental ability information of the person 10 with disabilities from the training device 20 or the rehabilitation facility apparatus 30 through the network N. Alternatively, the acquisition unit 522 may receive input of the physical and mental ability information by user operation through an input device directly connected to the retrieval apparatus 50 or a computer connected through a LAN, etc.

Further, the acquisition unit 522 may acquire, from each of a plurality of training devices used for training, the physical and mental ability information stored in each of the training devices. For example, when one person 10 with disabilities undergoes training by using a plurality of training devices 20, training results, physical evaluation values, and so on can be stored dispersedly in these training devices in some cases. Further, training results, physical evaluation values, and so on when a plurality of persons 10 with disabilities undergo training by using the same or a plurality of different training devices 20 can be stored dispersedly in these training devices in some cases. In such cases, the acquisition unit 522 may collect training results, physical evaluation values, and so on from the plurality of training devices 20, and accumulate them into the storage unit 51. In this case, the storage unit 51 may be used for various kinds of analysis, maintenance of the job definition information 511, and so on as a database of the physical and mental ability information.

Further, the acquisition unit 522 may acquire the current value of physical evaluation determined based on a result of training as the physical and mental ability information, and further acquire a target value of physical evaluation when the person 10 with disabilities is at work. Alternatively, the acquisition unit 522 may acquire a plurality of measured values by the training device 20 used for training as the physical and mental ability information.

Note that the acquisition unit 522 may collect, i.e., acquire, the physical and mental ability information at regular intervals, and accumulate them in association with the identification information of the person 10 with disabilities into the storage unit 51. Alternatively, the physical and mental ability information collected at regular intervals may be accumulated in an external database. In such cases, the acquisition unit 522 acquires the physical and mental ability information associated with the identification information of the person 10 with disabilities from the storage unit 51 or the external database.

The retrieval unit 523 retrieves (identifies), as potential jobs, the job information 5111 associated with the required ability information 5112 where the physical and mental ability information acquired by the acquisition unit 522 is taken into consideration based on the job definition information 511. At this time, the retrieval unit 523 according to this embodiment derives the deficiency of ability in the physical and mental ability information from the required ability information 5112, identifies supplementary information for supplementing the deficiency of ability, and retrieves a potential job by further taking the identified supplementary information into consideration in the physical and mental ability information. Note that the retrieval unit 523 does not necessarily derive the deficiency of ability, identify the supplementary information, and so on. For example, the retrieval unit 523 may derive the deficiency of ability, identify the supplementary information, etc. when the search condition received by the receiving unit 521 contains "a request for an assist device".

The retrieval unit 523 according to this embodiment identifies, as the supplementary information, one or more assist devices 5121 that can supplement the deficiency of ability of the person 10 with disabilities. Particularly, the retrieval unit 523 identifies the supplementary information (the assist device 5121) associated with the supplementary value 5122 that can supplement the deficiency of ability based on the supplementary definition information 512. Further, the retrieval unit 523 may identify, as the supplementary information, a plurality of types of assist devices 5121 that can supplement the deficiency of ability.

When retrieving a potential job, the retrieval unit 523 preferably retrieves, as the potential job, the job information 5111 further associated with the working environment 5113 that is appropriate for the acquired physical and mental ability information based on the job definition information 511. For example, the retrieval unit 523 may identify the required ability information 5112 that meets the acquired physical and mental ability information, further identifies the working environment 5113 to which the person is adaptable with his/her physical and mental ability information based on the job definition information 511, and determines the job information 5111 associated with the identified required ability information 5112 and the identified working environment 5113 as the potential job.

The calculation unit 524 calculates employment costs for the acquired physical and mental ability information and the retrieved potential job. It is assumed that the employment costs include, for the person 10 with disabilities, compensation such as wages (hourly pay, monthly pay, annual pay, etc.) when employed at the potential job, and include, for an employer, labor costs including wages needed when employing the person 10 with disabilities. Note that the calculation unit 524 may distinguish the employment costs to be calculated between wages etc. and labor costs depending on whether the job search request is made from the disability person 10 side or the employer side.

Employment costs may be calculated as follows, though not limited thereto. For example, a conversion map where employment costs are associated with each combination of the physical and mental ability information and a potential job may be stored in the storage unit 51, and the calculation unit 524 may calculate employment costs by using this conversion map. Alternatively, the calculation unit 524 may calculate employment costs by using a predetermined calculation algorithm from the physical and mental ability information and a potential job.

Further, as described above, when the retrieval unit 523 identifies a plurality of types of assist devices 5121, the calculation unit 524 may calculate a plurality of employment costs respectively corresponding to different combinations of the physical and mental ability information and the plurality of types of assist devices 5121.

The output unit 525 outputs presentation information based on a potential job. The presentation information is information for presenting information containing a potential job as a search result to a requestor of the job search request. For example, when a plurality of potential jobs are retrieved by the retrieval unit 523, the output unit 525 outputs a result of sorting these potential jobs in a specified order (a list of potential jobs) as the presentation information. Further, when the retrieval unit 523 identifies the supplementary information as described above, the output unit 525 outputs the presentation information containing the potential job and the supplementary information. The output unit 525 further adds the employment costs to the presentation information and outputs this information. Furthermore, when the calculation unit 524 calculates a plurality of employment costs as described above, the output unit 525 may further add the plurality of employment costs to the presentation information and outputs this information.

Note that each of the receiving unit 521, the acquisition unit 522, the retrieval unit 523, the calculation unit 524 and the output unit 525 described above may be implemented by dedicated hardware. Further, some or all of the elements of each device may be implemented by general-purpose or dedicated circuitry, processor, or a combination of them. They may be configured using a single chip, or a plurality of chips connected through a bus. Some or all of the elements of each device may be implemented by a combination of the above-described circuitry, etc. and a program. Further, a CPU (Central Processing Unit), a GPU (Graphics Processing Unit), an FPGA (field-programmable gate array), and so on may be used as a processor (the control unit 52).

In the case where some or all of the elements of the retrieval apparatus 50 are implemented by a plurality of information processing apparatuses, circuitries, and so on, the plurality of information processing apparatuses, circuitries, and so on may be centralized or dispersed. For example, the information processing apparatuses, circuitries, and so on may be implemented as a form in which components are connected through a communication network, such as a client-server system or a cloud computing system. Further, the functions of the retrieval apparatus 50 may be provided in a SaaS (Software as a Service) format.

The storage unit 51 may be an external storage device connected to the retrieval apparatus 50, and it may input and output data to and from the retrieval apparatus 50 by a storage system, a database system, etc. In this case, the external storage device at least stores the job definition information 511 and the supplementary definition information 512, and the storage unit 51 in the retrieval apparatus 50 may at least store the retrieval program 513.

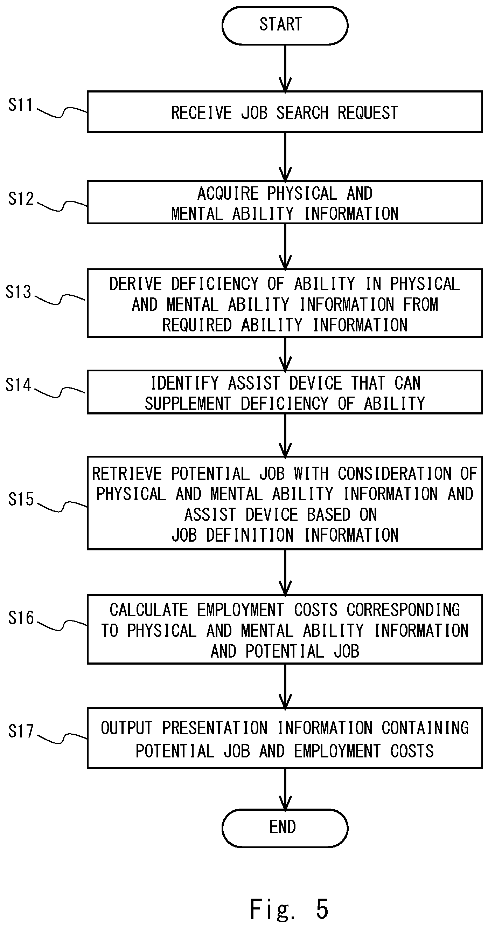

Fig. 5 is a flowchart showing the flow of a retrieval method according to the first embodiment. For example, a user on the disability person side enters a job search request containing the above-described search condition by using the disability person terminal 40 in order to find a workplace for employment for the person 10 with disabilities to be reintegrated into society, and the disability person terminal 40 transmits the entered job search request to the retrieval apparatus 50 through the network N. Alternatively, a user of the retrieval apparatus 50 enters the job search request by using an information processing apparatus connected to the retrieval apparatus 50 in response to a request from a user on the disability person side, etc., and this information processing apparatus outputs the entered search request to the retrieval apparatus 50.

The receiving unit 521 of the retrieval apparatus 50 receives the job search request from an information terminal or an information processing apparatus such as the disability person terminal 40 or the rehabilitation facility apparatus 30 described above (S11). It is assumed that the job search request at least contains the identification information of the person 10 with disabilities, and the job tasks desired by the person 10 with disabilities, and a request for an assist device as the above-described search condition. The job search request may further contain other search condition described above.

Next, the acquisition unit 522 of the retrieval apparatus 50 acquires the physical and mental ability information corresponding to the identification information of the person 10 with disabilities contained in the job search request (S12). For example, the acquisition unit 522 transmits an acquisition request for the physical and mental ability information that corresponds to the identification information of the person 10 with disabilities to the training device 20 or the rehabilitation facility apparatus 30 through the network N. The acquisition unit 522 then receives the physical and mental ability information transmitted from the training device 20 or the rehabilitation facility apparatus 30 through the network N in response to the acquisition request. Note that, as described above, when the physical and mental ability information is accumulated in the storage unit 51 or an external database, the acquisition unit 522 acquires the physical and mental ability information associated with the identification information of the person 10 with disabilities from the storage unit 51 or the external database.

Because the search condition contain "a request for an assist device", the retrieval unit 523 refers to the job definition information 511 and derives the deficiency of ability in the physical and mental ability information from the required ability information 5112 (S13). For example, the retrieval unit 523 may calculate a difference in ability value between each of the required ability information 5112 in the job definition information 511 and the physical and mental ability information of the person 10 with disabilities acquired in Step S12, and use the calculated difference as the deficiency of ability.

Then, the retrieval unit 523 identifies, as the supplementary information, one or more assist devices 5121 that can supplement the deficiency of ability of the person 10 with disabilities (S14). For example, the retrieval unit 523 identifies the supplementary value 5122 that exceeds the deficiency of ability from the supplementary definition information 512, and then identifies the assist device 5121 associated with this supplementary value 5122.

Then, the retrieval unit 523 retrieves a potential job with consideration of the physical and mental ability information of the person 10 with disabilities and the assist device 5121 identified in Step S14 based on the job definition information 511 (S15). For example, the retrieval unit 523 acquires the supplementary value 5122 associated with the assist device 5121 identified in Step S14 from the supplementary definition information 512. Then, the retrieval unit 523 calculates the supplemented physical and mental ability information by adding the acquired supplementary value 5122 to the physical and mental ability information of the person 10 with disabilities. After that, the retrieval unit 523 identifies the required ability information 5112 that meets the supplemented physical and mental ability information from the job definition information 511. Further, the retrieval unit 523 identifies the working environment 5113 to which the person is adaptable with the supplemented physical and mental ability information from the job definition information 511. For example, when the person 10 with disabilities assumes a use of a wheelchair from the physical and mental ability information of the person 10 with disabilities acquired in Step S12, or when the assist device 5121 identified in Step S14 is a wheelchair, the working environment 5113 to which the person is adaptable with the supplemented physical and mental ability information is such a place where there is a wheelchair-accessible toilet near the place of work. After that, the retrieval unit 523 retrieves the job information 5111 associated with the identified required ability information 5112 and the identified working environment 5113 as the potential job from the job definition information 511.

Note that, because the search condition contain job tasks (job conditions) desired by the person 10 with disabilities, the retrieval unit 523 narrows down the job information 5111 in the job definition information 511 to those matching the job conditions. For example, the retrieval unit 523 may narrow down the job information 5111 in the job definition information 511 to those matching the job conditions before Step S13, and then perform Steps S13 to S15. Alternatively, in Step S15, the retrieval unit 523 may retrieve the job information 5111 that meets the job conditions in addition to the required ability information 5112 and the working environment 5113 as the potential job.

After that, the calculation unit 524 calculates the employment costs for the physical and mental ability information acquired in Step S12 and the potential job retrieved in Step S15 (S16). In this example, it is assumed that the job search request is made from the disability person 10 side, and the calculation unit 524 calculates wages, etc. as the employment costs.

Then, the output unit 525 outputs presentation information that contains the potential job and the employment costs (S17). For example, the output unit 525 transmits the presentation information to the disability person terminal 40 that has made the job search request through the network N. A user on the disability person side can thereby obtain job information appropriate to the physical and mental ability information of the person 10 with disabilities.

As described above, according to this embodiment, it is possible to find an appropriate workplace that allows a person with disabilities to use his/her ability and thereby support his/her reintegration into society. Further, a company offering a job can employ a person with the required ability on the basis of job tasks. Further, there are cases where a subsidy for employment of people with disabilities is granted. Furthermore, a manufacturer that handles an assist device can promote the widespread use of their assist device.

Particularly, by identifying an assist device that can supplement the deficiency of ability and searching for a potential job with this assist device taken into consideration, even if the current physical and mental ability information does not meet the physical and mental ability required for a job, it is possible to present a potential job at which a person with disabilities can gain employment by working with an assist device. It is thereby possible to increase the choices of potential jobs.

Further, by calculating employment costs, it is possible to support determination on selecting a potential job more appropriately.

Generally, a job at which a person with disabilities can gain employment and their labor costs are different depending on the grade of disability. For example, it is assumed that the labor costs for employment of a person without disabilities is 300,000 yen, the labor costs for employment of a person with the 1st grade of disability is 200,000 yen, and the labor costs for employment of a person with the 2nd grade (which is a lower level of disability than the 1st grade) of disability is 250,000 yen. It is also assumed that, a specific job can be done by a person without disabilities or a person with the 2nd grade of disability, but is difficult to be done with the physical and mental ability of a person with the 1st grade of disability. In this case, a wheelchair that can supplement the deficiency of ability of the person with the 1st grade of disability is identified in this embodiment, and this person can do this job with use of the wheelchair. A person in human resources then sees the presentation information to which the above-described labor costs are added, and knows that a person with the 1st grade of disability can do this job by using a wheelchair as the assist device, and their labor costs are lower than those of a person without disabilities or a person with the 2nd grade of disability. Thus, this embodiment promotes the employment of people with disabilities.

### <Second Embodiment>

The second embodiment is a modified example of the above-described first embodiment. Note that the configuration of a retrieval system according to the second embodiment is the same as that of Fig. 1, and therefore the illustration of the same is omitted, and the description of the common elements is also omitted.

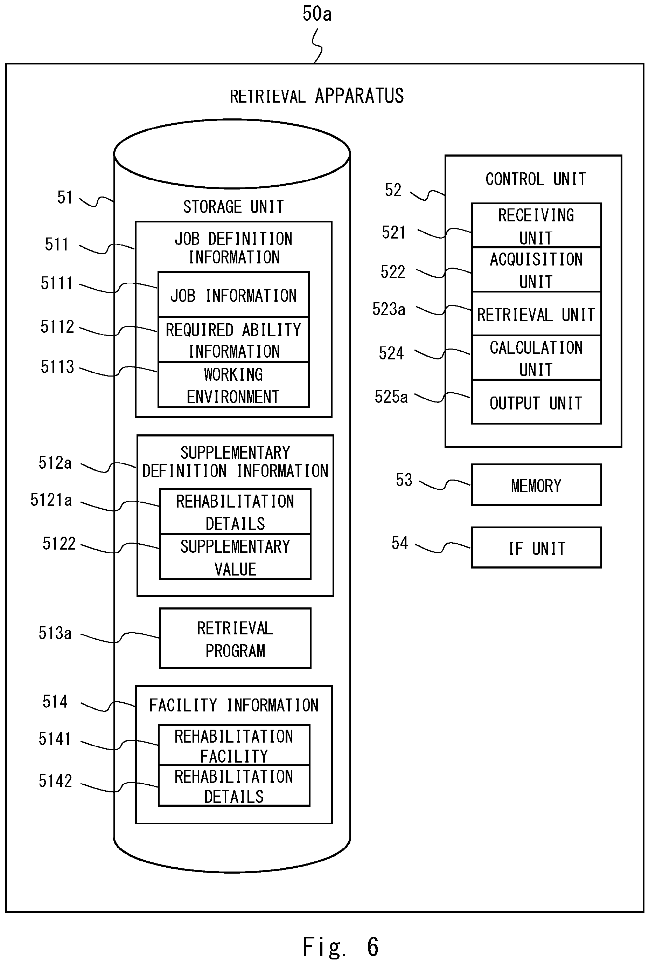

Fig. 6 is a block diagram showing the configuration of a retrieval apparatus 50a according to the second embodiment. Fig. 6 is different from Fig. 2 in that the supplementary definition information 512, the retrieval program 513, the retrieval unit 523 and the output unit 525 are replaced with supplementary definition information 512a, a retrieval program 513a, a retrieval unit 523a and an output unit 525a, and facility information 514 is added. The other elements are the same as those of the first embodiment, and the description of the common elements is omitted.

The supplementary definition information 512a associates rehabilitation details 5121a, which are an example of supplementary information, with a supplementary value 5122. The rehabilitation details 5121a are the details of training in order to improve the deficient ability. For example, the rehabilitation details 5121a indicate the type of training, such as gait training or independent movement with a wheelchair. A certain type of training in the rehabilitation details 5121a may be classified by details of training, a specific body part to be rehabilitated (thigh, knee, etc.), a training period, and so on. In this case, each rehabilitation details 5121a are associated with a different supplementary value 5122 depending on the details of training, a body part to be rehabilitated, or a training period in the gait training.

The retrieval program 513a is a computer program that performs processing for a retrieval method according to the second embodiment.

The facility information 514 is a database related to rehabilitation facilities, and, for example, where a rehabilitation facility 5141 and rehabilitation details 5142 are associated with each other. The rehabilitation facility 5141 is information about a facility, such as the identification information, name, address, etc. of the facility. The rehabilitation details 5142 are the identification information of the above-described rehabilitation details 5121a.

When the retrieval unit 523a identifies the supplementary information associated with the supplementary value 5122 that can supplement the deficiency of ability based on the supplementary definition information 512, it identifies, as the supplementary information, the rehabilitation details 5121a for improving the ability related to the deficiency of ability. Further, the retrieval unit 523a identifies the rehabilitation facility 5141 corresponding to the identified rehabilitation details 5142 (5121a) by referring to the facility information 514.

The output unit 525a adds the rehabilitation details and the rehabilitation facility to the potential job and the employment costs and presents them as the presentation information.

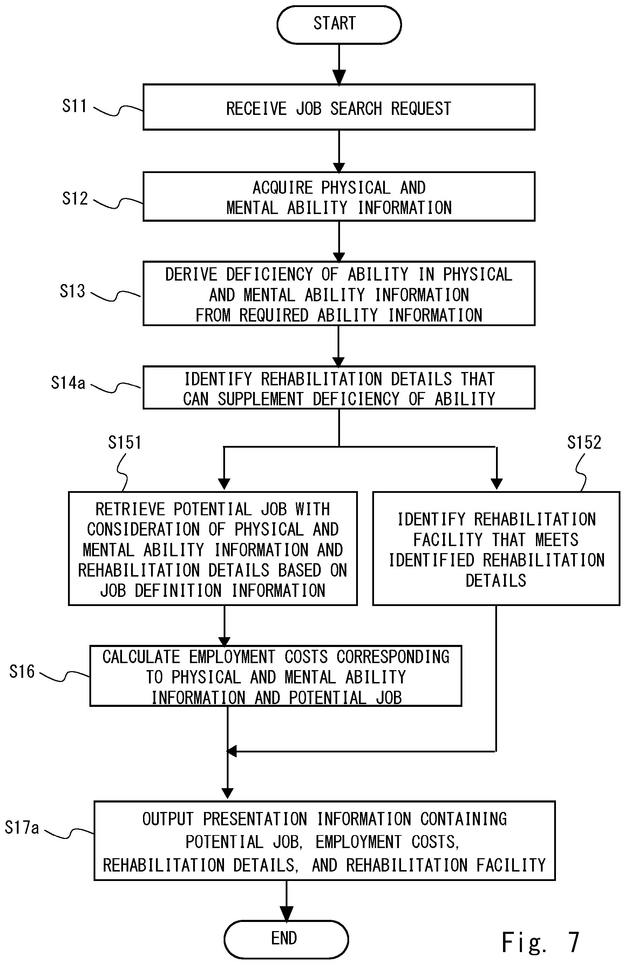

Fig. 7 is a flowchart showing the flow of a retrieval method according to the second embodiment. Fig. 7 is different from Fig. 5 in that Steps S14, S15, and S17 are replaced with S14a, S151 and S152, and S17a. The other steps are the same as those of Fig. 5, and the description thereof is omitted as appropriate.

After Step S13, the retrieval unit 523a identifies, as the supplementary information, the rehabilitation details 5121a that can supplement the deficiency of ability of the person 10 with disabilities (S14a). For example, the retrieval unit 523a identifies the supplementary value 5122 that exceeds the deficiency of ability among the supplementary definition information 512a, and then identifies the rehabilitation details 5121a associated with this supplementary value 5122.

Next, the retrieval unit 523a retrieves a potential job with consideration of the physical and mental ability information of the person 10 with disabilities and the rehabilitation details 5121a identified in Step S14a based on the job definition information 511 (S151). For example, the retrieval unit 523a acquires the supplementary value 5122 associated with the rehabilitation details 5121a identified in Step S14a from the supplementary definition information 512a. Then, the retrieval unit 523a calculates the supplemented physical and mental ability information where the acquired supplementary value 5122 is added to the physical and mental ability information of the person 10 with disabilities. After that, the retrieval unit 523a retrieves a potential job by using the supplemented physical and mental ability information in the same manner as in the above-described Step S15.

Then, the calculation unit 524 calculates the employment costs for the physical and mental ability information acquired in Step S12 and the potential job retrieved in Step S15a (S16).

Further, independently of Step S151, the retrieval unit 523a identifies the rehabilitation facility 5141 associated with the rehabilitation details 5121a identified in Step S14a from the facility information 514 (S152).

After Step S16 and S152, the output unit 525a outputs the presentation information to which the rehabilitation details 5121a identified in Step S14a and the rehabilitation facility 5141 identified in Step S152 are added in addition to the potential job and the employment costs (S17a).

As described above, according to this embodiment, even if the current physical and mental ability information does not meet the physical and mental ability required for a job, it is possible to search for a potential job by the improved physical and mental ability assumed to be achieved after a period of training indicated by the rehabilitation details has passed. It is thereby possible to increase the choices of potential jobs.

For example, it is effective in the case where the gait ability is improved by performing rehabilitation for gait training before the start of employment at a job, and the physical and mental ability required for this job can be satisfied after the rehabilitation. It is also effective in the case where the gait ability is gradually improved by starting rehabilitation for gait training in parallel with the start of employment at a job, and the physical and mental ability required for this job can be satisfied after a period of training has passed. Further, in this case, a company that employs a person with disabilities can expect that this person will become able to move independently while assistance with regard to walking is needed at the start of employment, and it is thereby possible to promote the employment of people with disabilities.

Further, by identifying and presenting the rehabilitation facility 5141 where training indicated by the rehabilitation details 5121a can be performed, the person 10 with disabilities can easily start the training indicated by the rehabilitation details 5121a.

Note that some rehabilitation facilities offer rehabilitation specific to a certain task, which is not covered by insurance. The facility information 514 may contain such rehabilitation facilities for patients to undergo rehabilitation at their own expense. In this case, the calculation unit 524 may further calculate rehabilitation costs when performing rehabilitation indicated by the rehabilitation details in the identified rehabilitation facility. Then, the output unit 525a may output the presentation information to which the rehabilitation costs are added. The person 10 with disabilities or a person on their side can thereby more appropriately select a potential job.

### <Third Embodiment>

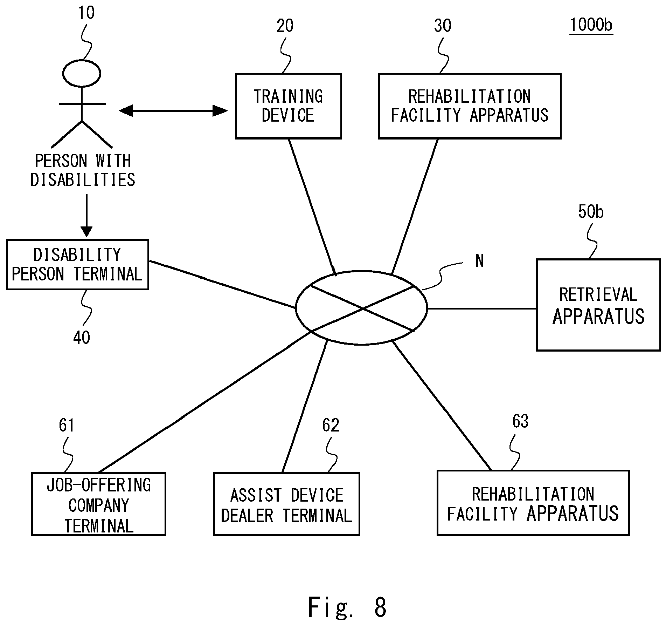

A third embodiment is a modified example of the above-described first or second embodiment. Fig. 8 is a block diagram showing the overall configuration of a retrieval system 1000b according to the third embodiment. Fig. 8 is different from Fig. 1 in that the retrieval apparatus 50 is replaced with a retrieval apparatus 50b, and a job-offering company terminal 61, an assist device dealer terminal 62, and a rehabilitation facility apparatus 63 are added. The other elements are the same as those of the first embodiment, and the description of the common elements is omitted.

The job-offering company terminal 61, the assist device dealer terminal 62, and the rehabilitation facility apparatus 63 are connected for communication with the retrieval apparatus 50b, etc. through a network N.

The job-offering company terminal 61 is an information processing apparatus to be used by a user who belongs to a company that is offering a job to employ a person with disabilities. The job-offering company terminal 61 has a communication function through the network N, and it may be a personal computer, or a portable information terminal such as a tablet terminal or a smartphone, for example. The job-offering company terminal 61 receives a job search request through an input device in response to operation by a user who belongs to this company, and transmits the job search request to the retrieval apparatus 50b through the network N. Further, the job-offering company terminal 61 receives the presentation information from the retrieval apparatus 50b through the network N, and outputs (displays etc.) this information to an output device such as a screen.

For example, in response to application for a job from the person 10 with disabilities, a person in human resources who belongs to this company may enter a job search request containing the identification information of the person 10 with disabilities and the job conditions as the search condition to the job-offering company terminal 61. Alternatively, when a job search request is transmitted from the disability person terminal 40 to the retrieval apparatus 50b, the job-offering company terminal 61 may receive the presentation information corresponding to this job search request from the retrieval apparatus 50b through the network N and display this information on a screen or the like.

The assist device dealer terminal 62 is an information processing apparatus to be used by a user who belongs to a dealer that handles (manufactures, delivers, etc.) at least a part of the above-described assist device 5121. The assist device dealer terminal 62 has a communication function through the network N, and it may be a personal computer, or a portable information terminal such as a tablet terminal or a smartphone, for example. The assist device dealer terminal 62, just like the disability person terminal 40 and the job-offering company terminal 61, can transmit a job search request to the retrieval apparatus 50b through the network N, receive the presentation information from the retrieval apparatus 50b through the network N, and output (displays etc.) this information to an output device such as a screen. Further, when a job search request is transmitted to the retrieval apparatus 50b from the disability person terminal 40 or the job-offering company terminal 61, the assist device dealer terminal 62 may receive the presentation information corresponding to this job search request from the retrieval apparatus 50b through the network N and display this information on a screen or the like.

Typically, however, the assist device dealer terminal 62 receives billing (a request for payment of a billed amount) from the retrieval apparatus 50b through the network N in some cases when the person 10 with disabilities who uses the assist device provided by this dealer is employed, as described later.

The rehabilitation facility apparatus 63 is, just like the rehabilitation facility apparatus 30, an information processing apparatus that is installed in a facility to perform rehabilitation or a facility that manages this rehabilitation, such as a medical institution, or that is at least administered by this facility. The training device 20 may be placed in this rehabilitation facility. The rehabilitation facility apparatus 63 has a communication function through the network N, and it may be a personal computer, or a portable information terminal such as a tablet terminal or a smartphone, for example. The rehabilitation facility apparatus 63, just like the disability person terminal 40 and the job-offering company terminal 61, can transmit a job search request to the retrieval apparatus 50b through the network N, receive the presentation information from the retrieval apparatus 50b through the network N, and output (displays etc.) this information to an output device such as a screen. Further, when a job search request is transmitted to the retrieval apparatus 50b from the disability person terminal 40 or the job-offering company terminal 61, the rehabilitation facility apparatus 63 may receive the presentation information corresponding to this job search request from the retrieval apparatus 50b through the network N and display this information on a screen or the like.

Typically, however, the rehabilitation facility apparatus 63 receives billing (a request for payment of a billed amount) from the retrieval apparatus 50b through the network N in some cases when the person 10 with disabilities is employed on condition that rehabilitation therapies that can be provided in the facility are performed in this facility, as described later.

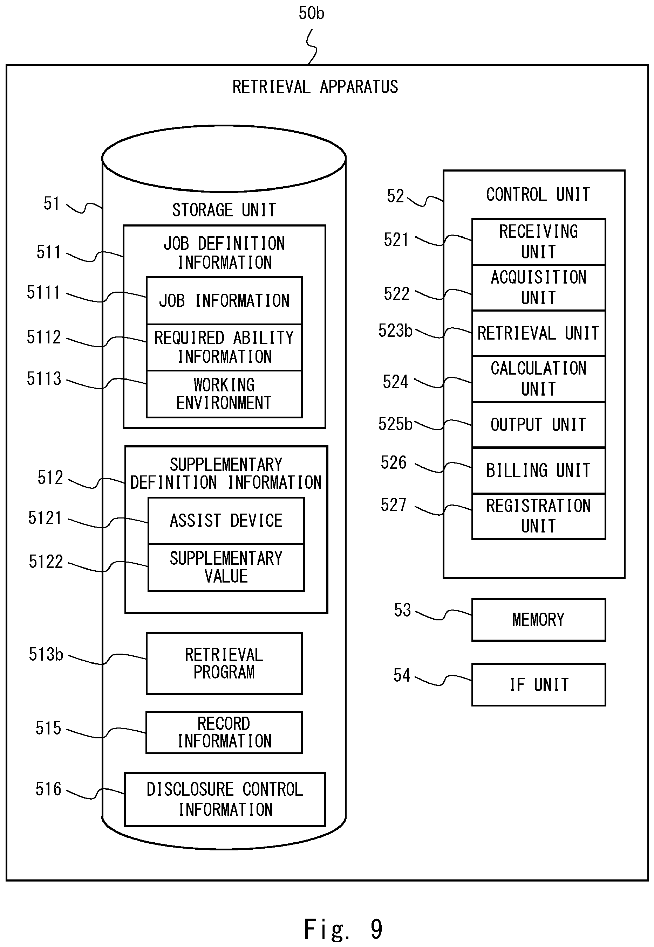

Fig. 9 is a block diagram showing the configuration of the retrieval apparatus 50b according to the third embodiment. Fig. 9 is different from Fig. 2 in that the retrieval program 513, the retrieval unit 523 and the output unit 525 are replaced with a retrieval program 513b, a retrieval unit 523b and an output unit 525b, and record information 515, disclosure control information 516, a billing unit 526 and a registration unit 527 are added. The other elements are the same as those of the first embodiment, and the description of the common elements is omitted. Further, the retrieval apparatus 50b may be created by adding the above-described modifications to the retrieval apparatus 50a according to the second embodiment. Alternatively, the retrieval apparatus 50b may be created by adding the above-described modifications to a device that integrates the retrieval apparatuses 50 and 50a.

The retrieval program 513b is a computer program that performs processing for a retrieval method according to the third embodiment.

The record information 515 is information that, when the person 10 with disabilities is employed at a potential job contained in the presentation information, associates this presentation information with the acquired physical and mental ability information. Further, in the record information 515, date and time of presentation, a receiver of presentation, the identification information of the person 10 with disabilities, and so on may be associated with each presentation information. Further, the record information 515 may be information that accumulates a record of the past presentation information regardless of whether the person 10 with disabilities gains employment or not. Note that the record information 515 may be stored in a storage device different from the storage unit 51 in the retrieval apparatus 50b, or in a database of record information such as an external storage device connected to the retrieval apparatus 50b through the network N.

The disclosure control information 516 is information for controlling the disclosure range of the presentation information. The disclosure control information 516 contains the definition of the disclosure range in accordance with a receiver of disclosure. For example, the disclosure control information 516 contains a receiver of disclosure, contents of disclosure, and so on. The receiver of disclosure may be a person with disabilities, a company offering a job, an assist device dealer, a rehabilitation facility, and so on, for example, which contains the address information of the disability person terminal 40, the job-offering company terminal 61, the assist device dealer terminal 62, and the rehabilitation facility apparatus 63. For example, when the receiver of disclosure is a person with disabilities or their party, the contents of disclosure may be job information for a potential job, the name of a company (when the company offering a job gives permission to disclose it), employment costs such as wages (not labor costs), at least a part of the physical and mental ability information of the person 10 with disabilities (information which a doctor etc. gives permission to disclose), the supplementary information (an assist device or the details of rehabilitation), and a rehabilitation facility. When the receiver of disclosure is a company offering a job, the contents of disclosure may be job information for a potential job, employment costs which are labor costs, at least a part of the physical and mental ability information of the person 10 with disabilities (information which a doctor etc. gives permission to disclose), the supplementary information (an assist device or the details of rehabilitation), and a rehabilitation facility. When the receiver of disclosure is an assist device dealer, the contents of disclosure may be job information for a potential job, at least a part of the physical and mental ability information of the person 10 with disabilities (information which a doctor etc. gives permission to disclose), and the supplementary information (an assist device or the details of rehabilitation). When the receiver of disclosure is a rehabilitation facility, the contents of disclosure may be job information for a potential job, at least a part of the physical and mental ability information of the person 10 with disabilities (information which a doctor etc. gives permission to disclose), and the supplementary information (an assist device or the details of rehabilitation). Note that, however, when the receiver of disclosure is a company offering a job, an assist device dealer, or a rehabilitation facility, personal information of the person 10 with disabilities may be excluded from the contents of disclosure.

Further, a contract for disclosing the presentation information may be made for each receiver of disclosure, and a disclosure range corresponding to fees for each contract may be defined. In this case, the disclosure control information 516 contains a receiver of disclosure, fees, and contents of disclosure. Alternatively, the disclosure control information 516 may be a pair of a receiver of disclosure and fees, and a fees/disclosure contents table that defines the association between fees and contents of disclosure may be used.

The retrieval unit 523b retrieves a potential job with the record information 515 also taken into consideration. Further, the retrieval unit 523b may identify the assist device that can supplement the deficiency of ability with the record information 515 also taken into consideration.

The output unit 525b outputs the presentation information whose disclosure range is varied depending on the receiver of disclosure. To be specific, the output unit 525b refers to the disclosure control information 516 and specifies the contents of disclosure for the requestor of the job search request as the receiver of disclosure, and then generates presentation information based on the specified contents of disclosure. The output unit 525b then transmits the generated presentation information to the address information of the receiver of disclosure through the network N. Alternatively, the output unit 525b may generate a plurality of presentation information respectively corresponding to the contents of disclosure for a plurality of receivers of disclosure contained in the disclosure control information 516, and transmit each of the generated plurality of presentation information to the address information of the corresponding receiver through the network N.

Further, the output unit 525b may output the presentation information whose disclosure range is varied depending on fees for a contract which a receiver of disclosure has made in order to receive disclosure of the presentation information. To be specific, the output unit 525b refers to the disclosure control information 516 and specifies the fees for the requestor of the job search request as the receiver of disclosure, and then refers to the above-described fees/disclosure contents table and specifies the contents of disclosure associated with the specified fees. Alternatively, the output unit 525b may specify the contents of disclosure corresponding to the fees for each of a plurality of receivers of disclosure contained in the disclosure control information 516.

When the person 10 with disabilities gains employment at the potential job contained in the presentation information (when employed by a company offering the job), the billing unit 526 bills a provider of the information contained in this presentation information. For example, the presentation information contains a potential job, an assist device, a rehabilitation facility, and so on. In this case, the billing unit 526 bills the company offering the job that has provided the potential job contained in the presentation information (which is, the company that has given employment). Further, the billing unit 526 bills the assist device dealer that has provided the assist device contained in the presentation information (which is the assist device to be used when the person 10 with disabilities is employed at the potential job). Furthermore, the billing unit 526 bills the rehabilitation facility contained in the presentation information (which is the rehabilitation facility to undergo rehabilitation when the person 10 with disabilities is employed at the potential job).

When the person 10 with disabilities gains employment at the potential job contained in the presentation information, the registration unit 527 generates record information by associating this presentation information with the acquired physical and mental ability information, and registers the record information into the storage unit 51.

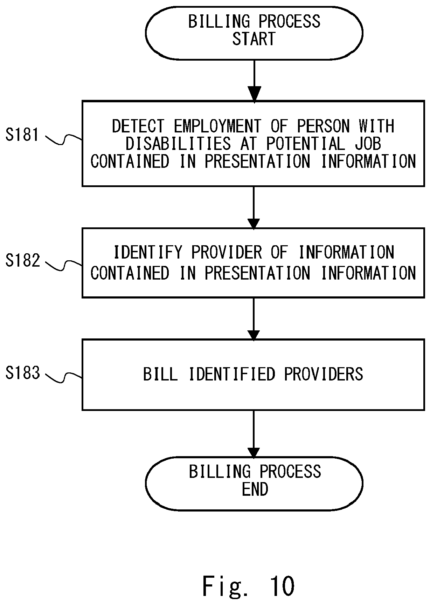

Fig. 10 is a flowchart showing the flow of a billing process according to the third embodiment. In this example, it is assumed that the person 10 with disabilities gains employment at one potential job in the presentation information, that is, the person 10 with disabilities is employed by a company offering a job based on information in the presentation information. In this case, the disability person terminal 40 sends notification of the potential job gained, the identification information of the company, the date and time the presentation information is output, the employment costs, and so on to the retrieval apparatus 50b through the network N in response to operation by a user on the disability person side. Alternatively, this notification may be sent from the job-offering company terminal 61 used by a person in human resources of this company. It is assumed that the retrieval apparatus 50b accumulates a record of combinations of the presentation information and the physical and mental ability information used for retrieval of information in this presentation information in the storage unit 51 or the like.

The billing unit 526 receives the above-described notification and thereby detects the employment of the person 10 with disabilities at the potential job contained in the presentation information (S181).

Then, the billing unit 526 identifies a provider of the information contained in the presentation information (S182). For example, the billing unit 526 refers to a record of the presentation information and specifies the presentation information and the potential job from the potential job, the identification information of the company, the date and time the presentation information is output, and so on contained in the notification. Then, the billing unit 526 identifies the assist device or the rehabilitation facility presented together with the specified potential job from the identified presentation information. After that, the billing unit 526 identifies the company offering a job and the assist device dealer, or the rehabilitation facility, which are providers of information about the identified potential job, assist device or rehabilitation facility.

Then, the billing unit 526 bills each of the identified providers (S183). For example, the billing unit 526 calculates a billing amount to each provider by a given calculation formula, etc. from the employment costs contained in the notification or the like. The billing unit 526 then stores billing management information containing the identification information of each provider and the billing amount into the storage unit 51. It is thereby possible to request payment of the billed amount to an operator of the job-offering company terminal 61, the assist device dealer terminal 62 or the rehabilitation facility apparatus 63 based on the billing management information by a billing system (not shown) etc. of the retrieval system 1000b. Note that the billing unit 526 may transmit a request for payment of a billed amount to the job-offering company terminal 61, the assist device dealer terminal 62 or the rehabilitation facility apparatus 63 through the network N.

As described above, by the billing process according to the third embodiment, it is possible to ensure the revenues of a job search service for people with disabilities.

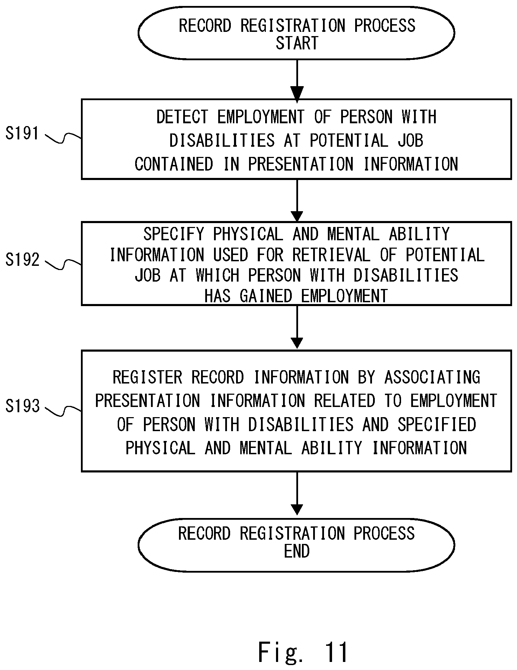

Fig. 11 is a flowchart showing the flow of a record registration process according to the third embodiment. First, the registration unit 527 receives the above-described notification and thereby detects the employment of the person 10 with disabilities at the potential job contained in the presentation information, just like in Step S181 (S191).

Next, the registration unit 527 specifies the physical and mental ability information used for retrieval of the potential job at which the person 10 with disabilities has gained employment (S192). For example, the registration unit 527 specifies the physical and mental ability information by referring to a record of combinations of the presentation information and the physical and mental ability information in the storage unit 51 or the like. Alternatively, when the physical and mental ability information is not accumulated in the storage unit 51 or the like, and the above-described notification contains the identification information of the person 10 with disabilities, the acquisition unit 522 may acquire the physical and mental ability information of the person 10 with disabilities contained in this notification as described above.

After that, the registration unit 527 generates record information by associating the presentation information related to employment of the person 10 with disabilities and the specified physical and mental ability information, and registers the record information in the storage unit 51 (S193).

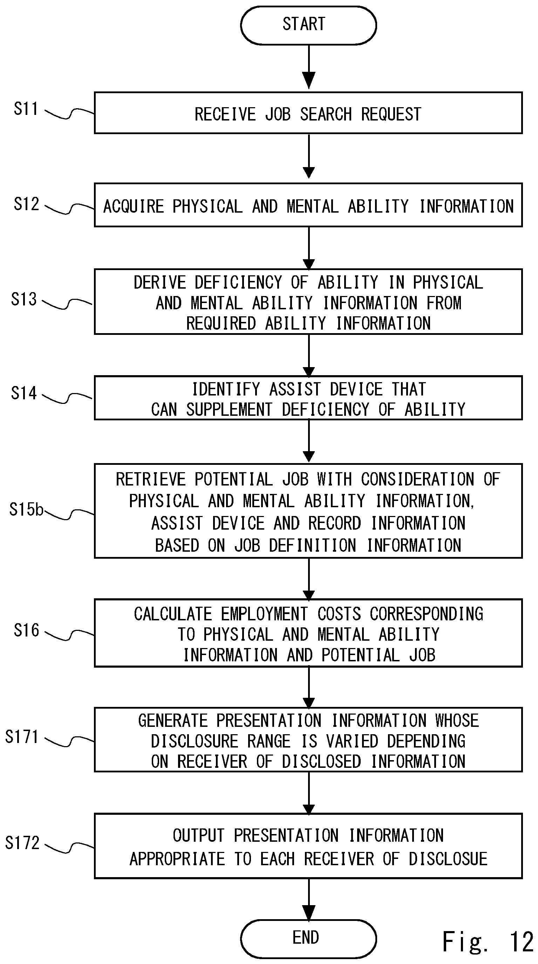

Fig. 12 is a flowchart showing the flow of a retrieval method according to the third embodiment. Fig. 12 is different from Fig. 5 in that Steps S15 and S17 are replaced with S15b and S171 and S172. The other steps are the same as those of Fig. 5, and the description thereof is omitted as appropriate. Alternatively, Steps S151 and S17a in Fig. 7 may be replaced with S15b and S171 and S172.

After Step S14, the retrieval unit 523b retrieves a potential job with the physical and mental ability information of the person 10 with disabilities, the assist device 5121 identified in Step S14, and the record information 515 also taken into consideration based on the job definition information 511 (S15b).

Further, after Step S16, the output unit 525b generates the presentation information whose disclosure range is varied depending on the receiver of disclosure (S171). The output unit 525b then outputs the presentation information appropriate to each receiver of disclosure (S172).

As described above, by taking the record information 515 accumulated by the record registration process according to the third embodiment into consideration at the time of search, it is possible to more accurately retrieve a potential job for the person 10 with disabilities having the physical and mental ability information similar to that in a record of past employment.

Further, by controlling the disclosure range depending on the receiver of disclosure according to the third embodiment, it is possible to provide appropriate information for each receiver and thereby allow each receiver to effectively use the information. In addition, it is possible to ensure the revenues of a job search service for people with disabilities by controlling the disclosure range depending on fees paid.

### <Fourth Embodiment>

A fourth embodiment is another example of the first to third embodiments described above. For staff assignment in a specific workplace, the fourth embodiment retrieves a job that can be assigned to a person with disabilities by taking the physical and mental ability information of the person into consideration.

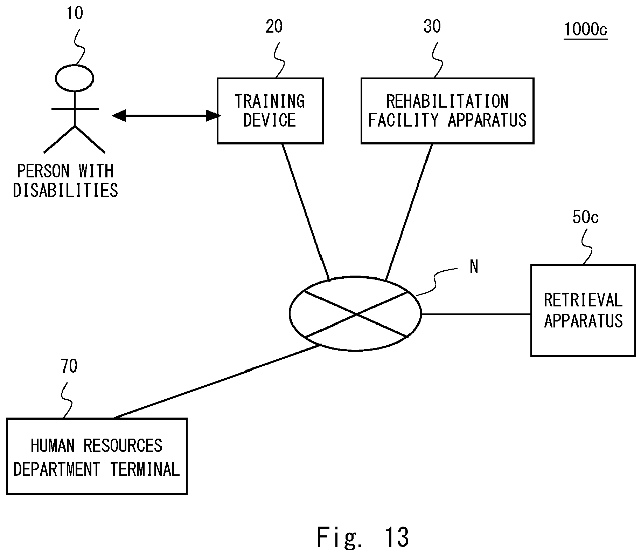

Fig. 13 is a block diagram showing the overall configuration of a retrieval system 1000c according to the fourth embodiment. Fig. 13 is different from Fig. 1 in that the retrieval apparatus 50 is replaced with a retrieval apparatus 50c, and a human resources department terminal 70 is added, and the disability person terminal 40 is eliminated. The other elements are the same as those of the first embodiment, and the description of the common elements is omitted.

The human resources department terminal 70 is connected for communication with the retrieval apparatus 50c, etc. through the network N. The human resources department terminal 70 is an information processing apparatus to be used by a human resources staff member who belongs to a human resources department of a company that employs the person 10 with disabilities. The job-offering company terminal 61 has a communication function through the network N, and it may be a personal computer, or a portable information terminal such as a tablet terminal or a smartphone, for example.

The human resources department terminal 70 receives a job search request to retrieve a job that is appropriate to the physical and mental ability of the person 10 with disabilities through an input device in response to operation of the human resources staff member, and transmits the job search request to the retrieval apparatus 50c through the network N. Further, the human resources department terminal 70 receives presentation information from the retrieval apparatus 50c through the network N, and outputs (displays etc.) the information to an output device such as a screen.

The retrieval apparatus 50c is the same as the retrieval apparatus 50 or 50a according to the first or second embodiment described above, and therefore the illustration and detailed description thereof are omitted. Note that, however, the job information 5111 according to the fourth embodiment is information about a department, a job category, and tasks to be assigned in a specific company. Further, the calculation unit 524 according to the fourth embodiment calculates labor costs for the person 10 with disabilities rather than employment costs.

In the fourth embodiment, when employing the person 10 with disabilities whose physical and mental ability information can be acquired, it is possible to assign tasks appropriate to the physical and mental ability information of the person 10 with disabilities, for example. Further, when changing staff positions on a regular basis or dynamically according to order situation, such as task assignment in a factory, it is possible to show appropriate options for possible positions for the person 10 with disabilities, together with positions for other workers. In this manner, the fourth embodiment can effectively support the determination or change of the job assignment, including the person 10 with disabilities whose physical and mental ability information can be acquired, in a company.

### <Other Embodiments>

The present disclosure does not necessarily use the supplementary definition information as described in the above embodiments. Specifically, the retrieval apparatus according to the present disclosure at least retrieves the job information where the acquired physical and mental ability information meets the required ability information as a potential job and presents this information. Alternatively, the retrieval apparatus according to the present disclosure may first retrieve the job information where the acquired physical and mental ability information meets the required ability information, and when a retrieval result shows no such information or less information than specified, it may identify the supplementary information that can supplement the deficiency of ability. Further, the retrieval apparatus according to the present disclosure may present both of a retrieval result when not using the supplementary information and a retrieval result when using the supplementary information.

The present disclosure may have the following configuration. Specifically, the acquisition unit may further acquire a dialogue history of the person with disabilities with a communication robot, and the retrieval unit may identify the supplementary information with this dialogue history also taken into consideration. The communication robot poses a question to the person 10 with disabilities in order to check the cognitive function, receives a response from the person 10 with disabilities, and accumulates the question and the response in association with time information as a dialogue history. Then, the retrieval apparatus may analyze the dialogue history acquired from the communication robot through the network N, determine the level of the cognitive function, derive the deficiency of ability based on the physical and mental ability information and this level, and identify the supplementary information as described above. It is thereby possible to more accurately retrieve a potential job that is appropriate to the cognitive function of the person 10 with disabilities.

Further, the acquisition unit may further acquire public subsidy information related to employment of people with disabilities, and the calculation unit may calculate the employment costs with this public subsidy information also taken into consideration. In employment of people with disabilities, the amount of subsidy provided is different depending on the grade of disability of a person with disabilities to be employed. Thus, when the grade of disability is contained in the physical and mental ability information of the person with disabilities to be employed, the calculation unit can calculate the substantial labor costs where the subsidy is subtracted from the labor costs by taking the public subsidy information into consideration. An employer can thereby more appropriately determine the employment of a person with disabilities, which promotes the employment of people with disabilities based on the substantial labor costs.

It should be noted that the present disclosure is not limited to the above-described embodiments and may be varied in many ways within the scope of the present disclosure. For example, although the present disclosure is described as a hardware configuration in the above embodiments, the present disclosure is not limited thereto. The present disclosure may be implemented by causing a CPU (Central Processing Unit) to execute a computer program to perform arbitrary processing.

In the above example, the program can be stored and provided to a computer using any type of non-transitory computer readable media. Non-transitory computer readable media include any type of tangible storage media. Examples of non-transitory computer readable media include magnetic storage media (such as floppy disks, magnetic tapes, hard disk drives, etc.), optical magnetic storage media (e.g. magneto-optical disks), CD-ROM (compact disc read only memory), CD-R (compact disc recordable), CD-R/W (compact disc rewritable), and semiconductor memories (such as mask ROM, PROM (programmable ROM), EPROM (erasable PROM), flash ROM, RAM (random access memory), etc.). The program may be provided to a computer using any type of transitory computer readable media. Examples of transitory computer readable media include electric signals, optical signals, and electromagnetic waves. Transitory computer readable media can provide the program to a computer via a wired communication line (e.g. electric wires, and optical fibers) or a wireless communication line.

From the disclosure thus described, it will be obvious that the embodiments of the disclosure may be varied in many ways. Such variations are not to be regarded as a departure from the spirit and scope of the disclosure, and all such modifications as would be obvious to one skilled in the art are intended for inclusion within the scope of the following claims.

## Claims

1. A retrieval system (1000) comprising:
a storage unit (51) configured to store job definition information associating job information with required ability information indicating physical and mental ability required for a job;
an acquisition unit (522) configured to acquire physical and mental ability information of a person with disabilities;
a retrieval unit (523) configured to retrieve, as a potential job, the job information associated with the required ability information in a case where the acquired physical and mental ability information is taken into consideration based on the job definition information; and
an output unit (525) configured to output presentation information based on the potential job.

2. The retrieval system (1000) according to Claim 1, wherein the physical and mental ability information contains a physical ability evaluation value based on a result of training performed by the person with disabilities to restore or maintain his/her physical ability by using a training device.

3. The retrieval system (1000) according to Claim 1 or 2, wherein
the retrieval unit (523) derives deficiency of ability in the physical and mental ability information from the required ability information, identifies supplementary information for supplementing the deficiency of ability, and retrieves the potential job, while also taking into consideration the identified supplementary information in the physical and mental ability information, and
the output unit (525) outputs the presentation information containing the potential job and the supplementary information.

4. The retrieval system (1000) according to Claim 3, wherein the retrieval unit (523) identifies, as the supplementary information, one or more assist devices that can supplement the deficiency of ability.

5. The retrieval system (1000) according to Claim 3 or 4, wherein the retrieval unit (523a) identifies, as the supplementary information, rehabilitation details for improving ability related to the deficiency of ability.

6. The retrieval system (1000) according to Claim 5, wherein
the retrieval unit (523a) identifies a rehabilitation facility that matches the identified rehabilitation details, and
the output unit (525a) outputs the presentation information to which the rehabilitation facility is added.

7. The retrieval system (1000) according to any one of Claims 3 to 6, wherein
the storage unit (51) further stores supplementary definition information associating each of a plurality of supplementary information with a supplementary value for improving physical and mental ability, and
the retrieval unit (523) identifies the supplementary information associated with the supplementary value that can supplement the deficiency of ability based on the supplementary definition information.

8. The retrieval system (1000) according to any one of Claims 3 to 7, wherein
the acquisition unit (522) further acquires a dialogue history of the person with disabilities with a communication robot, and
the retrieval unit (523) identifies the supplementary information, while also taking the dialogue history into consideration.

9. The retrieval system (1000) according to any one of Claims 1 to 8, further comprising:
a calculation unit (524) configured to calculate employment costs corresponding to the physical and mental ability information and the potential job,
wherein the output unit (525) outputs the presentation information to which the employment costs are added.

10. The retrieval system (1000) according to Claim 9, wherein
the acquisition unit (522) further acquires public subsidy information related to employment of people with disabilities, and
the calculation unit (524) calculates the employment costs, while also taking the public subsidy information into consideration.

11. The retrieval system (1000) according to Claim 4, wherein
the retrieval unit (523b) identifies, as the supplementary information, a plurality of types of assist devices that can supplement the deficiency of ability,
the retrieval system (1000) further comprises a calculation unit (524) configured to calculate a plurality of employment costs respectively corresponding to different combinations of the physical and mental ability information and each of the plurality of types of assist devices, and
the output unit (525b) outputs the presentation information to which the plurality of employment costs are added.

12. The retrieval system (1000) according to any one of Claims 1 to 11, wherein
working environment for a job is further associated with the job information in the job definition information, and
the retrieval unit (523b) retrieves, as the potential job, the job information further associated with the working environment appropriate to the physical and mental ability information.

13. The retrieval system (1000) according to any one of Claims 1 to 12, wherein the output unit (525b) outputs the presentation information whose disclosure range is varied depending on a receiver of disclosure.

14. A retrieval method, in a computer, comprising:
acquiring physical and mental ability information of a person with disabilities;
based on job definition information associating job information with required ability information indicating physical and mental ability required for a job, retrieving, as a potential job, the job information associated with the required ability information in a case where the acquired physical and mental ability information is taken into consideration; and
outputting presentation information based on the potential job.

15. A retrieval program causing a computer to execute:
acquiring physical and mental ability information of a person with disabilities;
based on job definition information associating job information with required ability information indicating physical and mental ability required for a job, retrieving, as a potential job, the job information associated with the required ability information in a case where the acquired physical and mental ability information is taken into consideration; and
outputting presentation information based on the potential job.
